# EUROPEAN PATENT SPECIFICATION

(11) **EP 0 546 563 B1**
(45) Date of publication and mention of the grant of the patent: **08.11.2000**
(21) Application number: 92121149.6
(22) Date of filing: 11.12.1992
(51) Int. Cl.: C07D 471/04, C07D 219/02, C07D 221/12

(54) **Complex crystal**
Komplex Kristall
Complexe cristallin

(30) Priority: 12.12.1991 JP 32911591; 14.07.1992 JP 18702092; 04.12.1992 JP 32559292
(43) Date of publication of application: 16.06.1993
(73) Proprietor: TOYOTA JIDOSHA KABUSHIKI KAISHA, Aichi-ken (JP)
(72) Inventor: Usuki, Arimitsu, c/o K.K. Toyota Chuo Kenkyusho, Aichi-gun, Aichi-ken (JP); Takeuchi, Hisato, c/o K.K. Toyota Chuo Kenkyusho, Aichi-gun, Aichi-ken (JP); Tatsuda, Narihito, c/o K.K. Toyota Chuo Kenkyusho, Aichi-gun, Aichi-ken (JP); Okada, Akane, c/o K.K. Toyota Chuo Kenkyusho, Aichi-gun, Aichi-ken (JP); Kurauchi, Toshio, c/o K.K. Toyota Chuo Kenkyusho, Aichi-gun, Aichi-ken (JP); Okayama, Shinobu, c/o K.K. Toyota Chuo Kenkyusho, Toyota-shi, Aichi-ken (JP); Tojima, Kazuo, c/o Toyota Jidosha Kabushiki Kaisha, Toyota-shi, Aichi-ken (JP)
(74) Representative: Bühling, Gerhard, Dipl.-Chem.

(56) References cited:
- GB-A- 1 586 127
- US-A- 4 131 334

## Description

### BACKGROUND OF THE INVENTION

### Field of the Invention

The present invention relates to a complex crystal which is suitable for use as a light-adjusting component of a light valve or a light-adjusting glass, and which is composed of a polycyclic aromatic compound and iodine.

### Description of the Related Art

Conventionally, a light-adjusting component has been used to disperse light-polarizing particles into a dispersion medium, and to make them orient by applying an electric field on the dispersion medium. Furthermore, the light-adjusting component has been used to randomize the light-polarizing particles to control optical properties (light-transmitting and light-screening performance) of the dispersion medium. It is proposed that a complex ion such as herapathite is used as light-polarizing particles. When the light-polarizing particles are used for a light-adjusting component, a dispersion medium containing the light-polarizing particles is filled into a gap between a pair of transparent electrodes attached to a pair of transparent substrates. The dispersion medium may be filled into a micro cell in order to improve optical properties or safety of the light-adjusting component.

The above-mentioned dispersion medium containing the light-polarizing particles almost contains moisture. Therefore, molecules of water destroy a clathlate structure of a complex ion. As a result, polarizability deteriorates. The light-polarizing particles which are known at present have poor moisture resisting property. In order to obtain durability and stability of a light-adjusting component, light-polarizing particles require excellent moisture resisting property.

Since automobiles have been improved their performance, it is desired that a windshield glass can screen a light. Therefore, it is necessary to manufacture a light-screening glass as a light-adjusting component by using light-polarizing particles. When a light-screening glass is formed by a light-adjusting component comprising the above-described light-polarizing particles, a dispersion medium containing the light-polarizing particles should be sealed in the middle stage of manufacturing a laminated glass. However, the light-polarizing particles deteriorate or decompose when they are exposed to a temperature above 100°C. Therefore, the light-polarizing particles are not suitable for use as the laminated glass for automobiles which is produced at a temperature above 130°C.

In order to obtain a long-lived light-adjusting component, it is required to provide a complex crystal which shows excellent heat resistance and excellent moisture resisting property.

From GB-A-1,586,127, a perhalide of an alkaloid acid salt is known as useful complex salt in a light-polarizing material. Specifically, cinchonidine derivatives are disclosed.

Similarly, US-A-4,131,334 discloses a perhalide of dihydrocinchonidine as a light-polarizing material.

The compounds described in the two latter references are said to exhibit chemical and temperature resistance.

### SUMMARY OF THE INVENTION

Accordingly, it is an object of the present invention to provide a complex crystal which is suitable for a light-adjusting component.

A complex crystal according to the present invention is characterized in that it is composed of anion of triiodine and cation of a polycyclic aromatic compound containing at least one nitrogen atom and 3 aromatic condensed rings and further an anion of an acid. The polycyclic aromatic compound is reacted with acid and iodine, thereby obtaining the complex crystal as a crystal comprising cation and anion. The complex crystal shows stable and strong polarizability, so it is suitable for a light-adjusting component which exhibits excellent heat resistance and excellent moisture resisting property.

A polycyclic aromatic compound include a plurality of aromatic rings being unified and having one side in common. The polycyclic aromatic compound, for example, includes naphthalene in which two benzene rings are condensed.

As for the polycyclic aromatic compound according to the present invention, the number of aromatic condensed rings is more than that of naphthalene, that is, the number of aromatic condensed rings is 3. The polycyclic aromatic compound should have at least one nitrogen atom. In the polycyclic aromatic compound, a nitrogen atom may exist as a heterocyclic compound, or it may be combined with a substitutional group.

Since the number of aromatic condensed rings is 3, the polycyclic aromatic compound covers an iodide ion (I₃⁻), and it protects a complex crystal from an attack of water or oxygen, Furthermore, the polycyclic aromatic compound stabilizes the whole of atoms stereochemically. Therefore, the complex crystal improves its stability, heat resistance and moisture resisting property. A nitrogen atom is an active spot for producing a complex.

The polycyclic aromatic compound containing nitrogen, for example, includes acridine, phenanthridine, 1,7-phenanthroline, 1,10-phenanthroline, 4,7-phenanthroline, phenazine, phenoxazine, phenothiazine, carbazole, or imino stilbene. Furthermore, it also includes a polycyclic aromatic compound in which amino group, amide group, hydrazide group, imino group, or guanidyl group is combined as a substituent.

Moreover, the polycyclic aromatic compound may contain a halogen substituent, aliphatic hydrocarbon substituent having less than 10 carbon atoms, an aromatic hydrocarbon substituent, or their combination by way of sulfur or oxygen atom. The substituent which is combined by way of sulfur or oxygen, for example, includes methoxy group, ethoxy group, phenoxy group, methylthio group, ethylthio group, or phenylthio group.

Concerning the above-described substituent, when the number of carbon atoms is more than 10, the complex crystal deteriorates its stability, heat resistance and moisture resisting property.

In this complex crystal, it seems that polycyclic aromatic compound is charged with a positive electric charge, and iodine is charged with a negative electric charge. Therefore, when a substituent having an electron donativity is combined with the aromatic ring, it stabilizes a combination of the aromatic ring, and improves heat resistance.

The complex crystal according to the present invention may contain acid ion as one of structural elements. The acid contained in the complex crystal neutralizes basicity of a nitrogen atom contained in the polycyclic aromatic compound to form a neutral salt. The acid includes inorganic acid and organic acid. The inorganic acid, for example, includes hydrochloric acid, sulfuric acid, phosphoric acid, or hydroiodic acid. The organic acid, for example, includes sulfonic acid such as benzenesulfonic acid, toluenesulfonic acid, or methanesulfonic acid; monocarboxylic acid such as acetic acid, propionic acid, butylic acid, valeric acid, trifluoroacetic acid, or benzoic acid; dicarboxylic acid such as oxalic acid, malonic acid, maleic acid, fumaric acid, or phthalic acid. The acid ion which is suitable for forming the complex crystal as a light-adjusting component is desirably sulfonic acid ion or dicarboxylic acid ion.

Ionized iodine forms a complex ion by being coordinated with an unsaturation system of the polycyclic aromatic compound. The complex crystal according to the present invention has a complex or coordinated structure in which a polycyclic aromatic compound and a molecular chain of iodine form a linear alternately and orderly. One surface of the polycyclic aromatic compound is parallel to the other surface. The complex crystal may have a clathlate structure.

The light-adjusting particles are used for a light valve or a light-adjusting glass, and they have light-screening performance and polarizability. The amount of light-screening, or polarizability can be adjusted by applying voltage. The light-adjusting particles according to the present invention have polarization, so they can be oriented by an action of an outer electric field. Furthermore, the light-adjusting particle has an acicular or a planar crystal structure. When the light-adjusting particles are dispersed randomly, they can screen a light. When the light-adjusting particles are oriented, they can transmit a light.

The complex crystal according to the present invention has polarizability by means of periodate contained in the complex crystal. Furthermore, the complex crystal has polarization by means of an interaction between the polycyclic aromatic compound and periodate. Therefore, the complex crystal can be used as light-adjusting particles for a light valve, a light-adjusting glass, a glare-proof mirror, a display component, or a light-polarizing component.

The above-described complex crystal is formed as follows. The afore-mentioned acid is added to the polycyclic aromatic compound to neutralize basicity of a nitrogen atom, thereby obtaining a solution of a neutral salt. A solution containing iodine and an iodate ion formed of a mixture of iodine and potassium iodate are added to the obtained solution of a neutral salt. As a result, the complex crystal is precipitated.

The obtained complex crystal is composed of the anion of triiodine formed from a mixture of iodine and potassium iodate and the cation of ionized polycyclic aromatic compound in which proton is added to a nitrogen atom. Therefore, the complex crystal is composed of a stable complex ion in which a charge of the cation of the polycyclic aromatic compound is neutralized with the anion of triiodine.

Concerning a polycyclic aromatic compound, an acid ion serves for forming the neutral salt in a solution, and iodine coordinates with the polycyclic aromatic compound. The iodine can combine one polycyclic aromatic compound with the other polycyclic aromatic compound to grow linearly the complex crystal. The complex crystal becomes large in an acicular or planar form, so it has a suitable size and form for a light-adjusting component.

The complex crystal has a stable structure because a polycyclic aromatic compound contains 3 aromatic condensed rings. Therefore, the complex crystal exhibits excellent heat resistance and excellent moisture resisting property which are suitable for a light-adjusting component.

The complex crystal according to the present invention has a structure in which cation of a polycyclic aromatic compound and a molecular chain of anion of triiodine are arranged alternately and orderly. Therefore, the complex crystal has polarizability because of an arrangement of the molecular chain of iodine.

As for the complex crystal, an electron placed on a complex unit is likely to move because of an interaction between the acid salt of polycyclic aromatic compound and iodate ion. Therefore, the complex crystal exhibits excellent polarization.

### BRIEF DESCRIPTION OF THE DRAWINGS

A more complete appreciation of the present invention and many of its advantages will be readily obtained as the same becomes better understood by reference to the following detailed description when considered in connection with the accompanying drawings and detailed specification, all of which forms apart of the disclosure:
Figure 1 shows a light-transmitting spectrum of a suspension of 1,10-phenanthroline periodate compound.
Figure 2 is a cross sectional view of an evaluation cell for showing a light-transmitting performance.
Figure 3 is a cross sectional view of an evaluation cell for showing a light-distransmitting performance.
Figure 4 is an X-ray diffraction chart of the complex crystal obtained in the First Preferred Embodiment.
Figure 5 is an X-ray diffraction chart of the complex crystal obtained in the Second Preferred Embodiment.
Figure 6 is an X-ray diffraction chart of the complex crystal obtained in the Third Preferred Embodiment.
Figure 7 is an X-ray diffraction chart of the complex crystal obtained in the Fourth Preferred Embodiment.
Figure 8 is an X-ray diffraction chart of the complex crystal obtained in the Fifth Preferred Embodiment.
Figure 9 is an X-ray diffraction chart of the complex crystal obtained in the Sixth Preferred Embodiment.
Figure 10 is an X-ray diffraction chart of the complex crystal obtained in the Seventh Preferred Embodiment.
Figure 11 is an X-ray diffraction chart of the complex crystal obtained in the Eighth Preferred Embodiment.
Figure 12 is an X-ray diffraction chart of the complex crystal obtained in the Ninth Preferred Embodiment.
Figure 13 is an X-ray diffraction chart of the complex crystal obtained in the Tenth Preferred Embodiment.
Figure 14 is an X-ray diffraction chart of the complex crystal obtained in the Eleventh Preferred Embodiment.

### DETAILED DESCRIPTION OF THE PREFERRED EMBODIMENTS

Having generally described the present invention, a further understanding can be obtained by reference to the specific preferred embodiments which are provided herein for purposes of illustration only and are not intended to limit the scope of the appended claims.

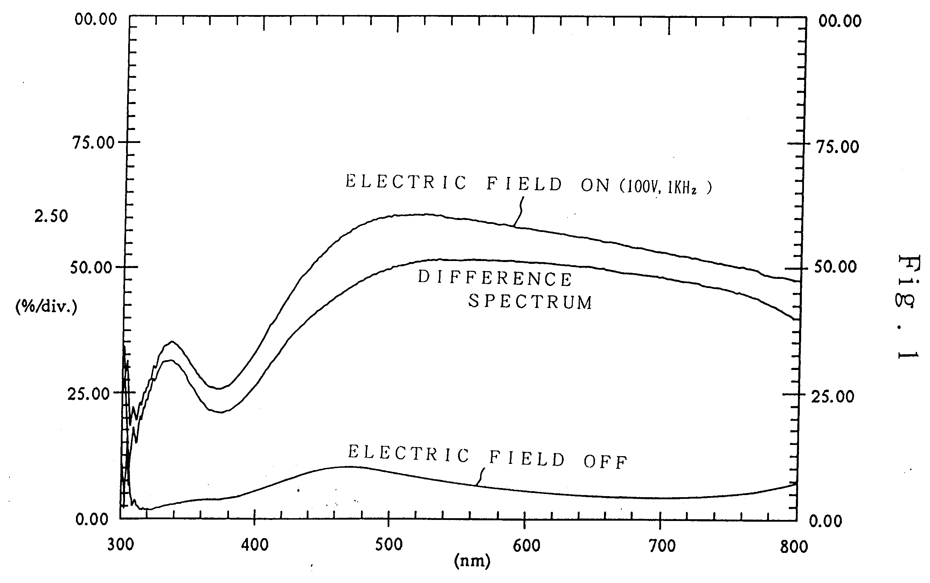

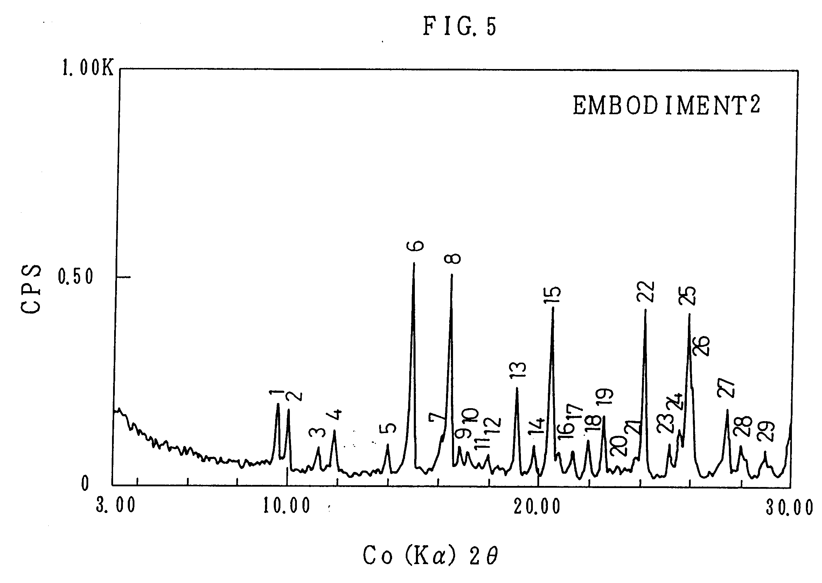

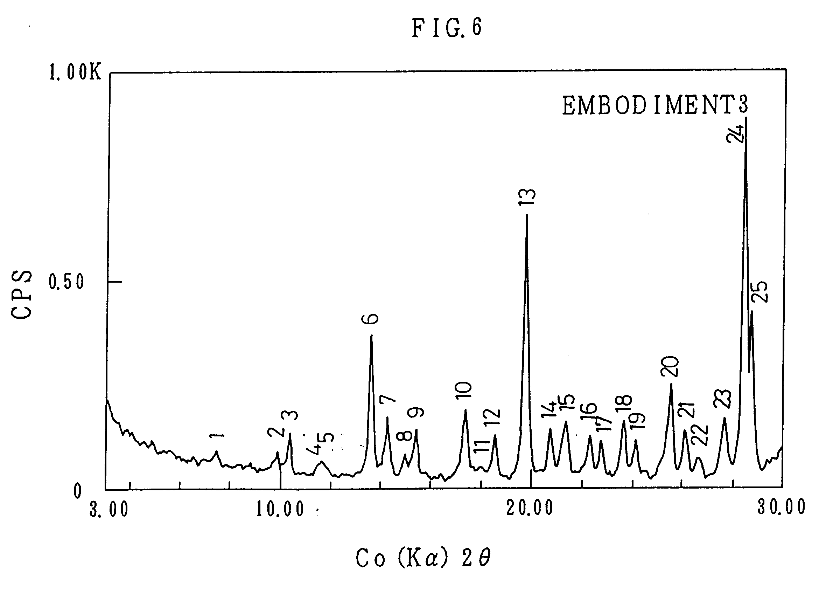

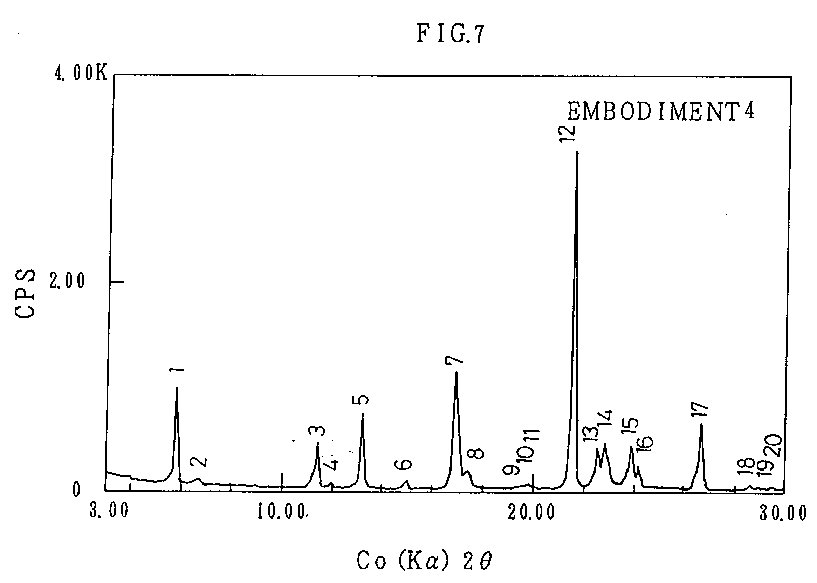

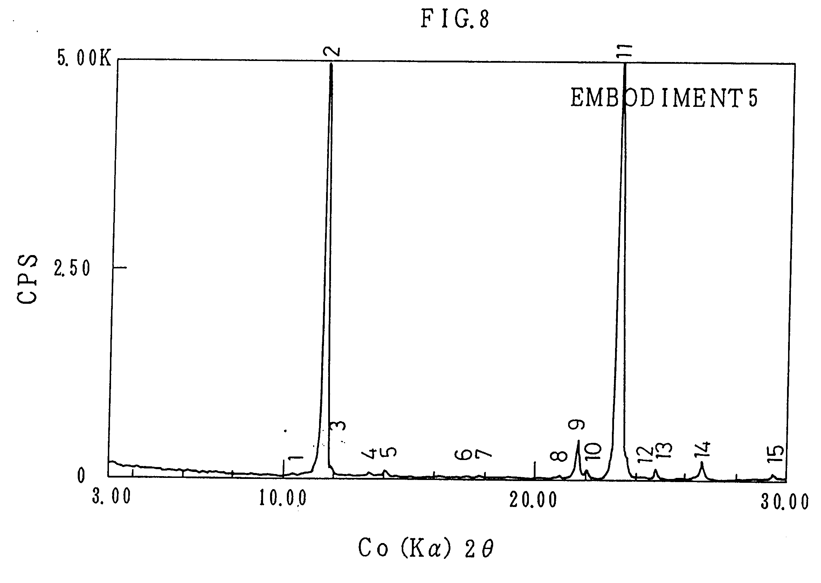

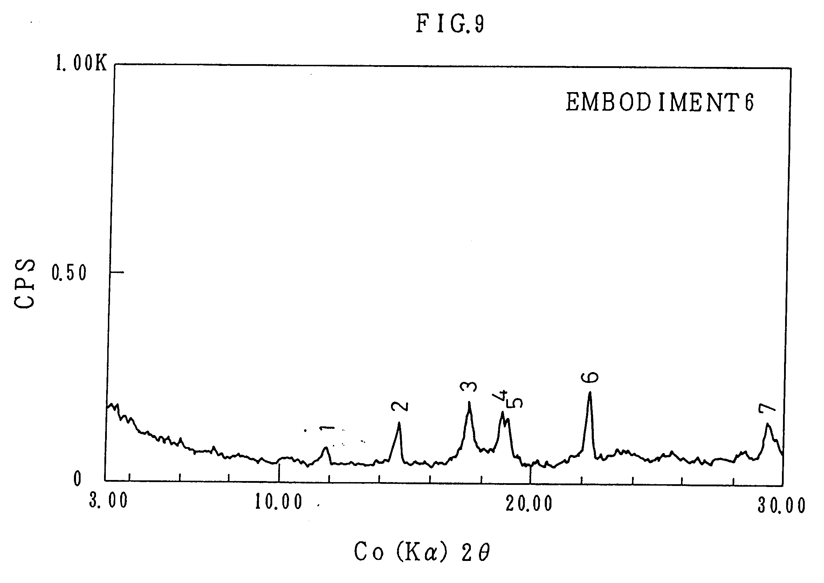

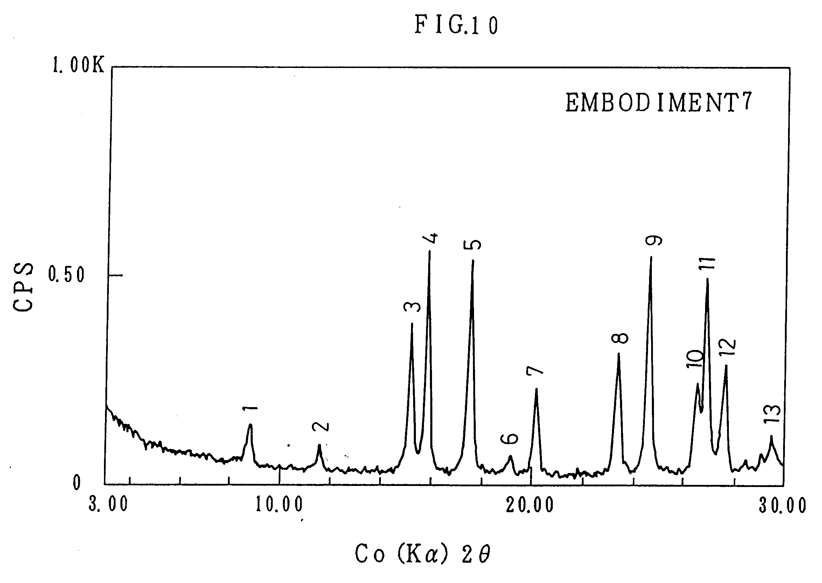

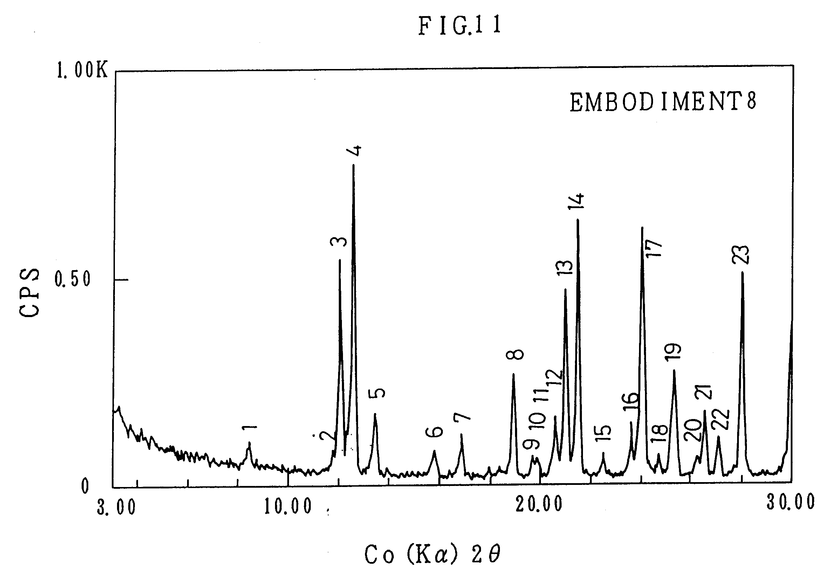

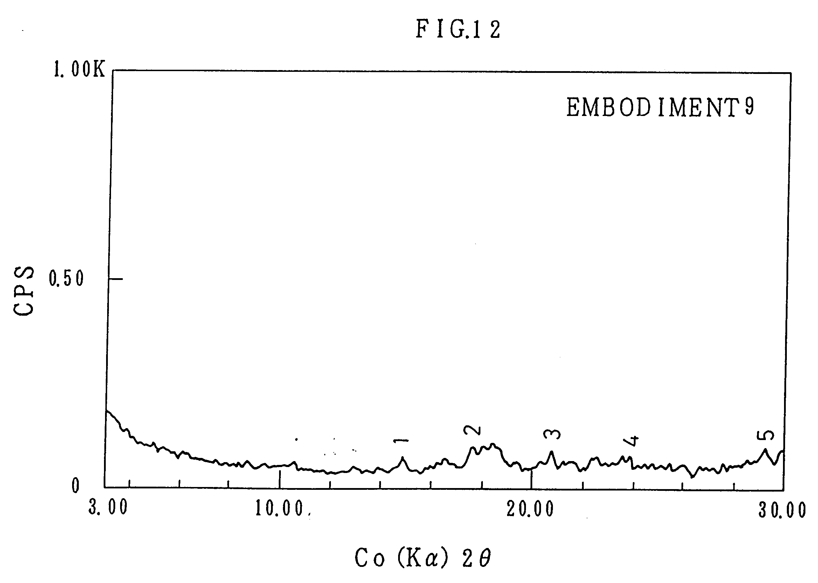

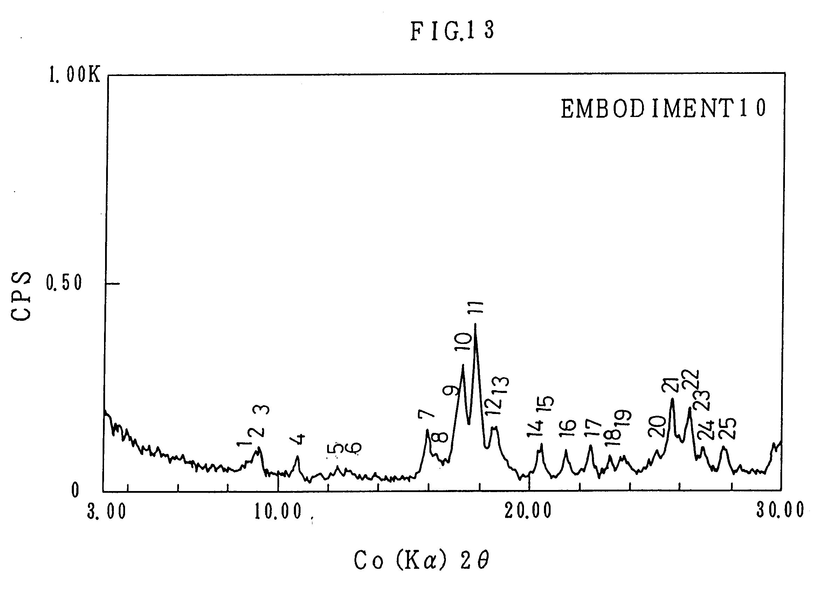

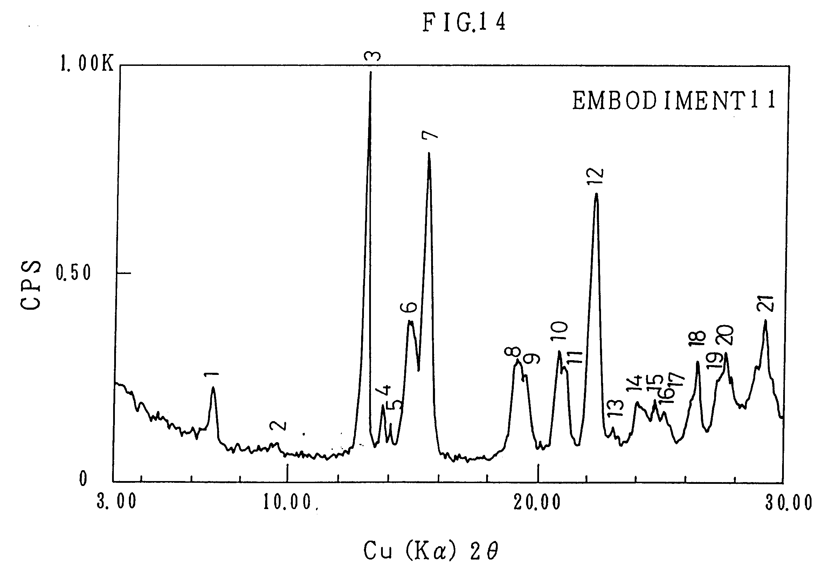

The Preferred Embodiments according to the present invention will be hereinafter described with reference to Figures 1 through 14.

### First Preferred Embodiment

A First Preferred Embodiment employed 1,7-phenanthroline to obtain a complex crystal comprising a 1,7-phenanthroline periodate compound as follows.

1.01g of 1,7-phenanthroline was dissolved into a mixed solution comprising 21g of water and 0.274g of concentrated sulfuric acid to prepare a first solution. 0.708g of iodine and 0.468g of potassium iodide were dissolved into a mixed solution comprising 14g of water and 3.5g of ethanol to prepare a second solution. The first and second solution were mixed and stirred for 1 hour to form a precipitate. The precipitate was filtered, washed, and vacuum dried to obtain 1.16g of a grayish-blue acicular crystal.

The crystal was identified as a complex crystal comprising a 1,7-phenanthroline periodate compound by means of an X-ray diffraction, an elemental analysis, a Raman spectrum, and an infrared-absorbing method. The complex crystal comprised 7 parts of a polycyclic aromatic compound, 4 parts of HI₃, and 1 part of H₂SO₄. The melting point of the complex crystal was 160.4°C according to a DSC measurement.

10g of ethanol was added to 0.2g of the complex crystal, and the mixture was purified by an ultrasonic cleaner for 10 minutes. The ethanol was removed from the complex crystal by a centrifugal separator. Furthermore, 10g of ditridecyl phthalate was added to 0.2g of the complex crystal, and the mixture was treated by the ultrasonic cleaner for 1 hour. The complex crystal was dispersed into ditridecyl phthalate to prepare a dispersion liquid. A volatile component such as remaining ethanol was removed from the liquid under a vacuum atmosphere, thereby obtaining a blue dispersed liquid in which the complex crystal comprising the 1,7-phenanthroline periodate compound was dispersed into ditridecyl phthalate. The color of the complex crystal never changed when the dispersed liquid was heated at a temperature of 130°C for 10 hours.

### Second Preferred Embodiment

A Second Preferred Embodiment employed 1,10-phenanthroline to obtain a complex crystal comprising a 1,10-phenanthroline periodate compound as follows.

1.01g of 1,10-phenanthroline was dissolved into a mixed solution comprising 21g of water and 0.274g of concentrated sulfuric acid to prepare a first solution. 0.708g of iodine and 0.468g of potassium iodide were dissolved into a mixed solution comprising 14g of water and 3.5g of ethanol to prepare a second solution. The first and second solution were mixed and stirred for 1 hour to form a precipitate. The precipitate was filtered, washed, and vacuum dried to obtain 1.60g of a brownish-red columnar crystal.

The crystal was identified as a complex crystal comprising a 1,10-phenanthroline periodate compound by means of an X-ray diffraction, an elemental analysis, a Raman spectrum, and an infrared-absorbing method. The complex crystal comprised 2 parts of a polycyclic aromatic compound, 1 part of HI₃, and 0.08 part of H₂SO₄. The melting point of the complex crystal was 171.1°C according to a DSC measurement.

10g of isopropanol was added to 0.2g of the complex crystal, and the mixture was purified by an ultrasonic cleaner for 10 minutes. The brownish-red columnar crystal changed to a blue fibrous crystal by means of the purification. The isopropanol was removed from the complex crystal by a centrifugal separator. Furthermore, 10g of ditridecyl phthalate was added to 0.2g of the complex crystal, and the mixture was treated by the ultrasonic cleaner for 1 hour. The complex crystal was dispersed into ditridecyl phthalate to prepare a dispersed liquid. A volatile component such as remaining isopropanol was removed from the liquid under a vacuum atmosphere, thereby obtaining a blue dispersed liquid in which the complex crystal comprising the 1,10-phenanthroline periodate compound was dispersed into ditridecyl phthalate. The color of the complex crystal never changed when the dispersed liquid was heated at a temperature of 130°C for 10 hours.

### Third Preferred Embodiment

A Third Preferred Embodiment employed 4,7-phenanthroline to obtain a complex crystal comprising a 4,7-phenanthroline periodate compound as follows.

1.01g of 4,7-phenanthroline was dissolved into a mixed solution comprising 21g of water and 0.274g of concentrated sulfuric acid to prepare a first solution. 0.708g of iodine and 0.468g of potassium iodide were dissolved into a mixed solution comprising 14g of water and 3.5g of ethanol to prepare a second solution. The first and second solution were mixed and stirred for 1 hour to form a precipitate. The precipitate was filtered, washed, and vacuum dried to obtain 1.60g of a brownish-black fibrous crystal.

The crystal was identified as a complex crystal comprising a 4,7-phenanthroline periodate compound by means of an X-ray diffraction, an elemental analysis, a Raman spectrum, and an infrared-absorbing method. The melting point of the complex crystal was 195.7°C according to a DSC measurement.

10g of ethanol was added to 0.2g of the complex crystal, and the mixture was purified by an ultrasonic cleaner for 10 minutes. The ethanol was removed from the complex crystal by a centrifugal separator. Furthermore, 10g of ditridecyl phthalate was added to 0.2g of the complex crystal, and the mixture was treated by the ultrasonic cleaner for 1 hour. The complex crystal was dispersed into ditridecyl phthalate to prepare a dispersed liquid. A volatile component such as remaining ethanol was removed from the liquid under a vacuum atmosphere, thereby obtaining a brownish-black dispersed liquid in which the complex crystal comprising the 4,7-phenanthroline periodate compound was dispersed into ditridecyl phthalate. The color of the complex crystal never changed when the dispersed liquid was heated at a temperature of 130°C for 10 hours.

### Fourth Preferred Embodiment

A Fourth Preferred Embodiment employed acridine to obtain a complex crystal comprising an acridine periodate compound as follows.

1.0g of acridine was dissolved into a mixed solution comprising 21g of water, 21g of ethanol, and 0.274g of concentrated sulfuric acid to prepare a first solution. 0.708g of iodine and 0.468g of potassium iodide were dissolved into a mixed solution comprising 14g of water and 3.5g of ethanol to prepare a second solution. The first and second solution were mixed and stirred for 1 hour to form a precipitate. The precipitate was filtered, washed, and vacuum dried to obtain 2.02g of a greenish-blue fibrous crystal.

The complex crystal was identified as a complex crystal comprising an acridine periodate compound by means of an X-ray diffraction, an elemental analysis, a Raman spectrum, and an infrared-absorbing method. The complex crystal comprised 4 parts of a polycyclic aromatic compound, 2 parts of HI₃, and 1 part of H₂SO₄. The melting point of the complex crystal was 204.8°C according to a DSC measurement.

10g of ethanol was added to 0.2g of the complex crystal, and the mixture was purified by an ultrasonic cleaner for 10 minutes. The ethanol was removed from the complex crystal by a centrifugal separator. Furthermore, 10g of ditridecyl phthalate was added to 0.2g of the complex crystal, and the mixture was treated by the ultrasonic cleaner for 1 hour. The complex crystal was dispersed into ditridecyl phthalate to prepare a dispersed liquid. A volatile component such as remaining ethanol was removed from the liquid under a vacuum atmosphere, thereby obtaining a greenish-blue dispersed liquid in which the complex crystal comprising the acridine periodate compound was dispersed into ditridecyl phthalate. The color of the complex crystal never changed when the dispersed liquid was heated at a temperature of 130°C for 10 hours.

### Fifth Preferred Embodiment

A Fifth Preferred Embodiment employed phenanthridine to obtain a complex crystal comprising a phenanthridine periodate compound as follows.

1.01g of phenanthridine was dissolved into a mixed solution comprising 21g of water and 0.274g of concentrated sulfuric acid to prepare a first solution. 0.708g of iodine and 0.468g of potassium iodide were dissolved into a mixed solution comprising 14g of water and 3.5g of ethanol to prepare a second solution. The first and second solution were mixed and stirred for 1 hour to form a precipitate. The precipitate was filtered, washed, and vacuum dried to obtain 1.22g of a brownish-black planar crystal.

The crystal was identified as a complex crystal comprising a phenanthridine periodate compound by means of an X-ray diffraction, an elemental analysis, a Raman spectrum, and an infrared-absorbing method. The melting point of the complex crystal was 185.1°C according to a DSC measurement.

10g of ethanol was added to 0.2g of the complex crystal, and the mixture was purified by an ultrasonic cleaner for 10 minutes. The ethanol was removed from the complex crystal by a centrifugal separator. Furthermore, 10g of ditridecyl phthalate was added to 0.2g of the complex crystal, and the mixture was treated by the ultrasonic cleaner for 1 hour. The complex crystal was dispersed into ditridecyl phthalate to prepare a dispersed liquid. A volatile component such as remaining ethanol was removed from the liquid under a vacuum atmosphere, thereby obtaining a dark-brown dispersed liquid in which the complex crystal comprising the phenanthridine periodate compound was dispersed into ditridecyl phthalate. The color of the complex crystal never changed when the dispersed liquid was heated at a temperature of 130°C for 10 hours.

### Sixth Preferred Embodiment

A Sixth Preferred Embodiment employed 4-methyl-1,10-phenanthroline to obtain a complex crystal comprising a 4-methyl-1,10-phenanthroline periodate compound as follows.

1.08g of 4-methyl-1,10-phenanthroline was dissolved into a mixed solution comprising 21g of water and 0.274g of concentrated sulfuric acid to prepare a first solution. 0.708g of iodine and 0.468g of potassium iodide were dissolved into a mixed solution comprising 14g of water and 3.5g of ethanol to prepare a second solution. The first and second solution were mixed and stirred for 1 hour to form a precipitate. The precipitate was filtered, washed, and vacuum dried to obtain 1.78g of a brownish-black granular crystal.

The crystal was identified as a complex crystal comprising a 4-methyl-1,10-phenanthroline periodate compound by means of an X-ray diffraction, an elemental analysis, a Raman spectrum, and an infrared-absorbing method. The melting point of the complex crystal was 215.2°C according to a DSC measurement.

10g of ethanol was added to 0.2g of the complex crystal, and the mixture was purified by an ultrasonic cleaner for 10 minutes. The ethanol was removed from the complex crystal by a centrifugal separator. Furthermore, 10g of ditridecyl phthalate was added to 0.2g of the complex crystal, and the mixture was treated by the ultrasonic cleaner for 1 hour. The complex crystal was dispersed into ditridecyl phthalate to prepare a dispersed liquid. A volatile component such as remaining ethanol was removed from the liquid under a vacuum atmosphere, thereby obtaining a brownish-black dispersed liquid in which the complex crystal comprising the 4-methyl-1,10-phenanthroline periodate compound was dispersed into ditridecyl phthalate. The color of the complex crystal never changed when the dispersed liquid was heated at a temperature of 130°C for 10 hours.

### Seventh Preferred Embodiment

A Seventh Preferred Embodiment employed 5-methyl-1,10-phenanthroline to obtain a complex crystal comprising a 5-methyl-1,10-phenanthroline periodate compound as follows.

1.08g of 5-methyl-1,10-phenanthroline was dissolved into a mixed solution comprising 21g of water and 0.274g of concentrated sulfuric acid to prepare a first solution. 0.708g of iodine and 0.468g of potassium iodide were dissolved into a mixed solution comprising 14g of water and 3.5g of ethanol to prepare a second solution. The first and second solution were mixed and stirred for 1 hour to form a precipitate. The precipitate was filtered, washed, and vacuum dried to obtain 2.03g of a dark-green acicular crystal.

The crystal was identified as a complex crystal comprising a 5-methyl-1,10-phenanthroline periodate compound by means of an X-ray diffraction, an elemental analysis, a Raman spectrum, and an infrared-absorbing method. The melting point of the complex crystal was 223.2°C according to a DSC measurement.

10g of ethanol was added to 0.2g of the complex crystal, and the mixture was purified by an ultrasonic cleaner for 10 minutes. The ethanol was removed from the complex crystal by a centrifugal separator. Furthermore, 10g of ditridecyl phthalate was added to 0.2g of the complex crystal, and the mixture was treated by the ultrasonic cleaner for 1 hour. The complex crystal was dispersed into ditridecyl phthalate to prepare a dispersed liquid. A volatile component such as remaining ethanol was removed from the liquid under a vacuum atmosphere, thereby obtaining a dark-green dispersed liquid in which the complex crystal comprising the 5-methyl-1,10-phenanthroline periodate compound was dispersed into ditridecyl phthalate. The color of the complex crystal never changed when the dispersed liquid was heated at a temperature of 130°C for 10 hours.

### Eighth Preferred Embodiment

An Eighth Preferred Embodiment employed 2,9-dimethyl-1,10-phenanthroline to obtain a complex crystal comprising a 2,9-dimethyl-1,10-phenanthroline periodate compound as follows.

1.16g of 2,9-dimethyl-1,10-phenanthroline was dissolved into a mixed solution comprising 21g of water and 0.274g of concentrated sulfuric acid to prepare a first solution. 0.708g of iodine and 0.468g of potassium iodide were dissolved into a mixed solution comprising 14g of water and 3.5g of ethanol to prepare a second solution. The first and second solution were mixed and stirred for 1 hour to form a precipitate. The precipitate was filtered, washed, and vacuum dried to obtain 1.65g of a brown granular crystal.

The crystal was identified as a complex crystal comprising a 2,9-dimethyl-1,10-phenanthroline periodate compound by means of an X-ray diffraction, an elemental analysis, a Raman spectrum, and an infrared-absorbing method. The melting point of the complex crystal was 153.8°C according to a DSC measurement.

10g of ethanol was added to 0.2g of the complex crystal, and the mixture was purified by an ultrasonic cleaner for 10 minutes. The ethanol was removed from the complex crystal by a centrifugal separator. Furthermore, 10g of ditridecyl phthalate was added to 0.2g of the complex crystal, and the mixture was treated by the ultrasonic cleaner for 1 hour. The complex crystal was dispersed into ditridecyl phthalate to prepare a dispersed liquid. A volatile component such as remaining ethanol was removed from the liquid under a vacuum atmosphere, thereby obtaining a brown dispersed liquid in which the complex crystal comprising the 2,9-dimethyl-1,10-phenanthroline periodate compound was dispersed into ditridecyl phthalate. The color of the complex crystal never changed when the dispersed liquid was heated at a temperature of 130°C for 10 hours.

### Ninth Preferred Embodiment

A Ninth Preferred Embodiment employed 4,7-dimethyl-1,10-phenanthroline to obtain a complex crystal comprising a 4,7-dimethyl-1,10-phenanthroline periodate compound as follows.

1.16g of 4,7-dimethyl-1,10-phenanthroline was dissolved into a mixed solution comprising 21g of water and 0.274g of concentrated sulfuric acid to prepare a first solution. 0.708g of iodine and 0.468g of potassium iodide were dissolved into a mixed solution comprising 14g of water and 3.5g of ethanol to prepare a second solution. The first and second solution were mixed and stirred for 1 hour to form a precipitate. The precipitate was filtered, washed, and vacuum dried to obtain 1.94g of a greenish-blue strip crystal.

The crystal was identified as a complex crystal comprising a 4,7-dimethyl-1,10-phenanthroline periodate compound by means of an X-ray diffraction, an elemental analysis, a Raman spectrum, and an infrared-absorbing method. The melting point of the complex crystal was 144.5°C according to a DSC measurement.

10g of ethanol was added to 0.2g of the complex crystal, and the mixture was purified by an ultrasonic cleaner for 10 minutes. The ethanol was removed from the complex crystal by a centrifugal separator. Furthermore, 10g of ditridecyl phthalate was added to 0.2g of the complex crystal, and the mixture was treated by the ultrasonic cleaner for 1 hour. The complex crystal was dispersed into ditridecyl phthalate to prepare a dispersed liquid. A volatile component such as remaining ethanol was removed from the liquid under a vacuum atmosphere, thereby obtaining a greenish-blue dispersed liquid in which the complex crystal comprising the 4,7-dimethyl-1,10-phenanthroline periodate compound was dispersed into ditridecyl phthalate. The color of the complex crystal never changed when the dispersed liquid was heated at a temperature of 130°C for 10 hours.

### Tenth Preferred Embodiment

A Tenth Preferred Embodiment employed 5,6-dimethyl-1,10-phenanthroline to obtain a complex crystal comprising a 5,6-dimethyl-1,10-phenanthroline periodate compound as follows.

1.16g of 5,6-dimethyl-1,10-phenanthroline was dissolved into a mixed solution comprising 21g of water and 0.274g of concentrated sulfuric acid to prepare a first solution. 0.708g of iodine and 0.468g of potassium iodide were dissolved into a mixed solution comprising 14g of water and 3.5g of ethanol to prepare a second solution. The first and second solution were mixed and stirred for 1 hour to form a precipitate. The precipitate was filtered, washed, and vacuum dried to obtain 1.94g of a red acicular crystal.

The crystal was identified as a complex crystal comprising a 5,6-dimethyl-1,10-phenanthroline periodate compound by means of an X-ray diffraction, an elemental analysis, a Raman spectrum, and an infrared-absorbing method. The melting point of the complex crystal was 206.9°C according to a DSC measurement.

10g of ethanol was added to 0.2g of the complex crystal, and the mixture was purified by an ultrasonic cleaner for 10 minutes. The ethanol was removed from the complex crystal by a centrifugal separator. Furthermore, 10g of ditridecyl phthalate was added to 0.2g of the complex crystal, and the mixture was treated by the ultrasonic cleaner for 1 hour. The complex crystal was dispersed into ditridecyl phthalate to prepare a dispersed liquid. A volatile component such as remaining ethanol was removed from the liquid under a vacuum atmosphere, thereby obtaining a red dispersed liquid in which the complex crystal comprising the 5,6-dimethyl-1,10-phenanthroline periodate compound was dispersed into ditridecyl phthalate. The color of the complex crystal never changed when the dispersed liquid was heated at a temperature of 130°C for 10 hours.

### Eleventh Preferred Embodiment

An Eleventh Preferred Embodiment employed 5-methoxy-1,10-phenanthroline to obtain a complex crystal comprising a 5-methoxy-1,10-phenanthroline periodate compound as follows.

1.27g of 5-methoxy-1,10-phenanthroline was dissolved into a mixed solution comprising 21g of water and 0.138g of concentrated sulfuric acid to prepare a first solution. 0.708g of iodine and 0.468g of potassium iodide were dissolved into a mixed solution comprising 14g of water and 3.5g of ethanol to prepare a second solution. The first and second solution were mixed and stirred for 1 hour to form a precipitate. The precipitate was filtered, washed, and vacuum dried to obtain 2.13g of a dark-green fibrous crystal.

The complex crystal was identified as a complex crystal comprising a 5-methoxy-1,10-phenanthroline periodate compound by means of an X-ray diffraction, an elemental analysis, a Raman spectrum, and an infrared-absorbing method. The melting point of the complex crystal was 229.5°C according to a DSC measurement. 10g of ethanol was added to 0.2g of the complex crystal, and the mixture was purified by an ultrasonic cleaner for 10 minutes. The ethanol was removed from the complex crystal by a centrifugal separator.

Furthermore, 10g of ditridecyl phthalate was added to 0.2g of the complex crystal, and the mixture was treated by the ultrasonic cleaner for 1 hour. The complex crystal was dispersed into ditridecyl phthalate to prepare a dispersed liquid. A volatile component such as remaining ethanol was removed from the liquid under a vacuum atmosphere, thereby obtaining a blue dispersed liquid in which the complex crystal comprising the 5-methoxy-1,10-phenanthroline periodate compound was dispersed into ditridecyl phthalate. The color of the complex crystal never changed when the dispersed liquid was heated at a temperature of 130°C for 10 hours.

### Twelfth Preferred Embodiment

A Twelfth Preferred Embodiment employed 5-amino-1,10-phenanthroline to obtain a complex crystal comprising a 5-amino-1,10-phenanthroline periodate compound as follows.

1.0g of 5-amino-1,10-phenanthroline was dissolved into a mixed solution comprising 50g of water, 10g of ethanol and 0.126g of concentrated sulfuric acid to prepare a first solution. 0.649g of iodine and 0.425g of potassium iodide were dissolved into a mixed solution comprising 12.8g of water and 3.26g of ethanol to prepare a second solution. The first and second solution were mixed and stirred for 1 hour to form a precipitate. The precipitate was filtered, washed, and vacuum dried to obtain 1.72g of a dark blue acicular crystal.

The crystal was identified as a complex crystal comprising a 5-amino-1,10-phenanthroline periodate compound by means of an X-ray diffraction, an elemental analysis, a Raman spectrum, and an infrared-absorbing method. The melting point of the complex crystal was 176.0°C according to a DSC measurement.

10g of ethanol was added to 0.2g of the complex crystal, and the mixture was purified by an ultrasonic cleaner for 10 minutes. The ethanol was removed from the complex crystal by a centrifugal separator. Furthermore, 10g of ditridecyl phthalate was added to 0.2g of the complex crystal, and the mixture was treated by the ultrasonic cleaner for 1 hour. The complex crystal was dispersed into ditridecyl phthalate to prepare a dispersed liquid. A volatile component such as remaining ethanol was removed from the liquid under a vacuum atmosphere, thereby obtaining a blue dispersed liquid in which the complex crystal comprising the 5-amino-1,10-phenanthroline periodate compound was dispersed into ditridecyl phthalate. The color of the complex crystal never changed when the dispersed liquid was heated at a temperature of 130°C for 10 hours.

### Thirteenth Preferred Embodiment

A Thirteenth Preferred Embodiment employed acridine to obtain a complex crystal comprising an acridine periodate compound as follows.

1.0g of acridine was dissolved into a mixed solution comprising 21g of water, 21g of ethanol, and 2.79mmol of one of organic acids shown in Table 4 to prepare a first solution. 0.708g of iodine and 0.468g of potassium iodide were dissolved into a mixed solution comprising 14g of water and 3.5g of ethanol to prepare a second solution. The first and second solution were mixed and stirred for 1 hour to form a precipitate. The precipitate was filtered, washed, and vacuum dried to obtain a complex crystal comprising an acridine periodate compound.

Other complex crystals comprising an acridine periodate compound are obtained by using each organic acids shown in Table 4. Table 5 shows the added amount, the yield, the color, and the shape of these complex crystals.

10g of isopropanol was added to 0.2g of the complex crystal, and the mixture was purified by an ultrasonic cleaner for 10 minutes. The isopropanol was removed from the complex crystal by a centrifugal separator. Furthermore, 10g of ditridecyl phthalate was added to 0.2g of the complex crystal, and the mixture was treated by the ultrasonic cleaner for 1 hour. The complex crystal was dispersed into ditridecyl phthalate to prepare a dispersed liquid. A volatile component such as remaining isopropanol was removed from the liquid under a vacuum atmosphere, thereby obtaining a brownish-black dispersed liquid in which the complex crystal comprising the acridine periodate compound was dispersed into ditridecyl phthalate. The color of the complex crystal never changed when the dispersed liquid was heated at a temperature of 130°C for 10 hours.

### Comparative Example

A Comparative Example employed dihydrocinchonidine comprising three nitrogen atoms and two aromatic compounds to obtain a complex crystal comprising a dihydrocinchonidine periodate compound according to the method disclosed in Japanese Unexamined Patent Publication (Kokai) No. 144893/1978.

As described in the Preferred Embodiments, the obtained complex crystal was dispersed into ditridecyl phthalate to prepare a dispersed liquid, and the liquid was heated at a temperature of 130°C for 10 hours. As a result, the color of the complex crystal changed at a temperature below 85°C, and it never restored at a room temperature.

Concerning each complex crystal according to the Preferred Embodiments, the color of the complex crystal never changed when the dispersed liquid in which the complex crystal was dispersed into ditridecyl phthalate was heated at a temperature of 130°C for 10 hours. Therefore, each complex crystal according to the Preferred Embodiments was stable, and it showed excellent heat resistance. However, the color of the dihydrocinchonidine periodate compound according to the Comparative Example changed at a temperature below 85°C. Therefore, the dihydrocinchonidine periodate compound exhibited poor heat resistance.

As for each dispersed liquid according to the Preferred Embodiments, 1g of the dispersed liquid was dripped into 10g of water, and stirred for 1 hour. The color of the complex crystal which was dispersed never changed when the dispersed liquid was mixed with water. Therefore, each complex crystal according to the Preferred Embodiments was stable, and it showed excellent moisture resisting property. On the contrary, the color of the dihydrocinchonidine periodate compound according to the Comparative Example changed to achromatic in a few minutes when the dispersed liquid in which the complex crystal comprising the dihydrocinchonidine periodate compound was dispersed into ditridecyl phthalate was dripped into water. Therefore, the dihydrocinchonidine periodate compound exhibited poor moisture resisting property.

Concerning the Preferred Embodiments 1 to 11, Tables 1 to 3 show the structural formula of the polycyclic aromatic compounds; the color, the shape, and the melting point of the complex crystals; the color of the dispersed liquid; heat resistance and moisture resisting property. As for the Preferred Embodiment 13, Table 4 shows organic acids, and Table 5 shows the added amount, the yield, the color, and the shape of the complex crystals.

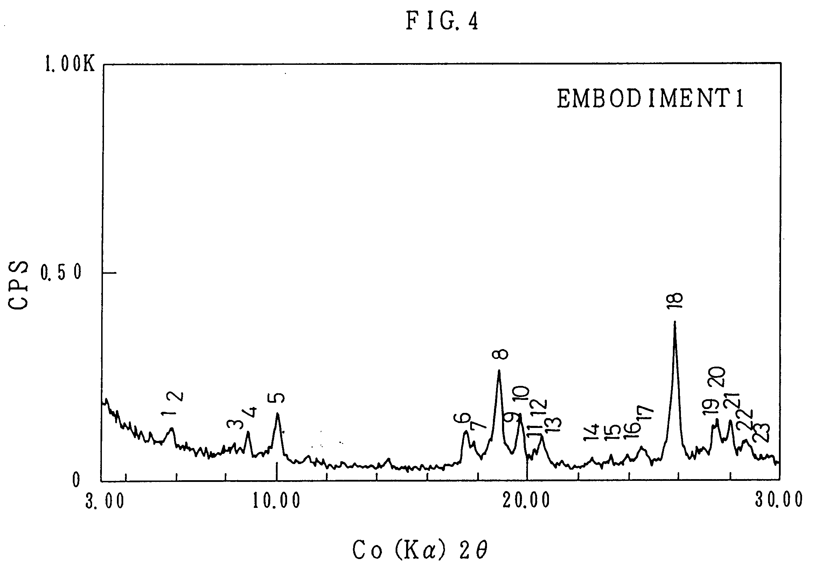

Table 6 shows the composition of elements of the complex crystals and the composition ratio of the complex crystals (polycyclic aromatic compound : HI₃ : H₂SO₄) obtained in the Preferred Embodiments 1,2,4,6,7, and 11. Furthermore, Figures 4 to 14 show the X-ray diffraction charts of the complex crystals obtained in the Preferred Embodiments 1 to 11.

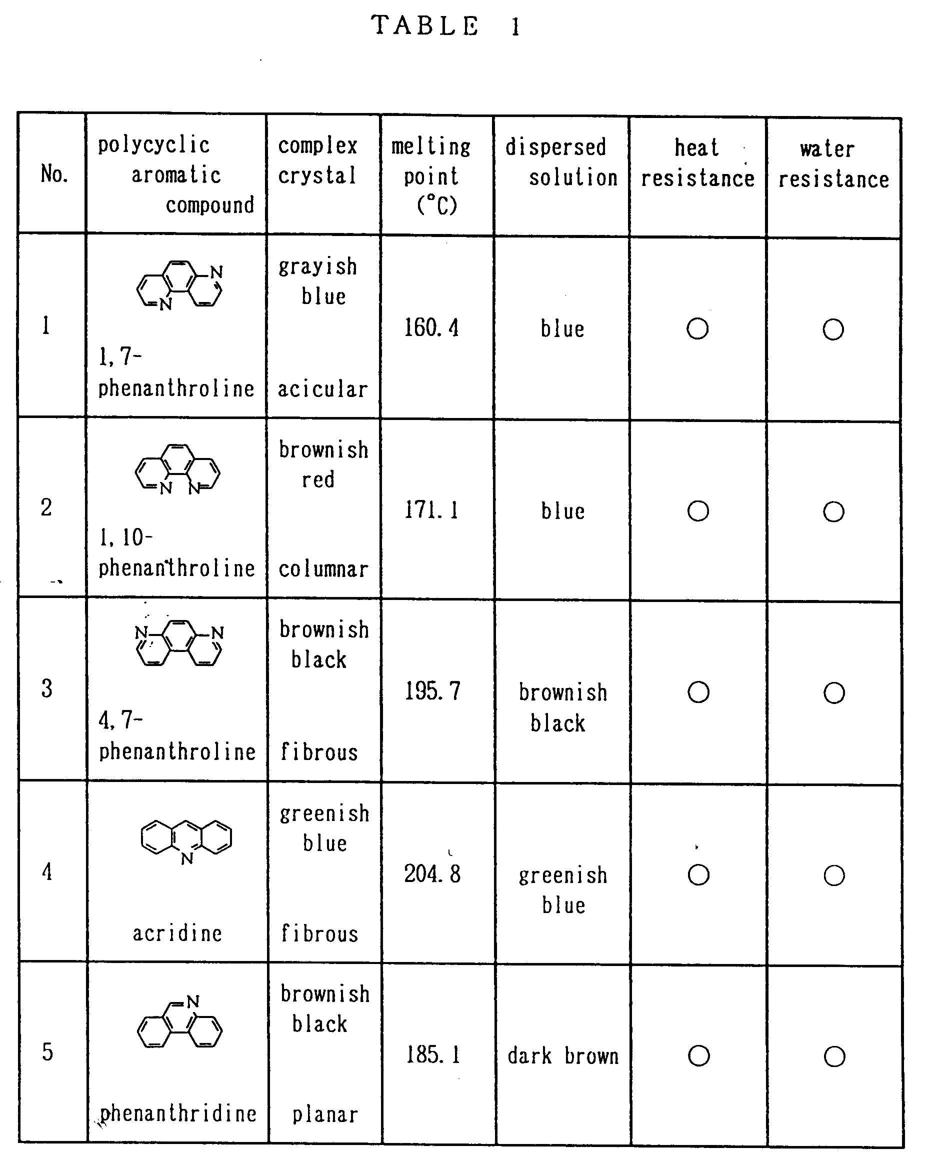

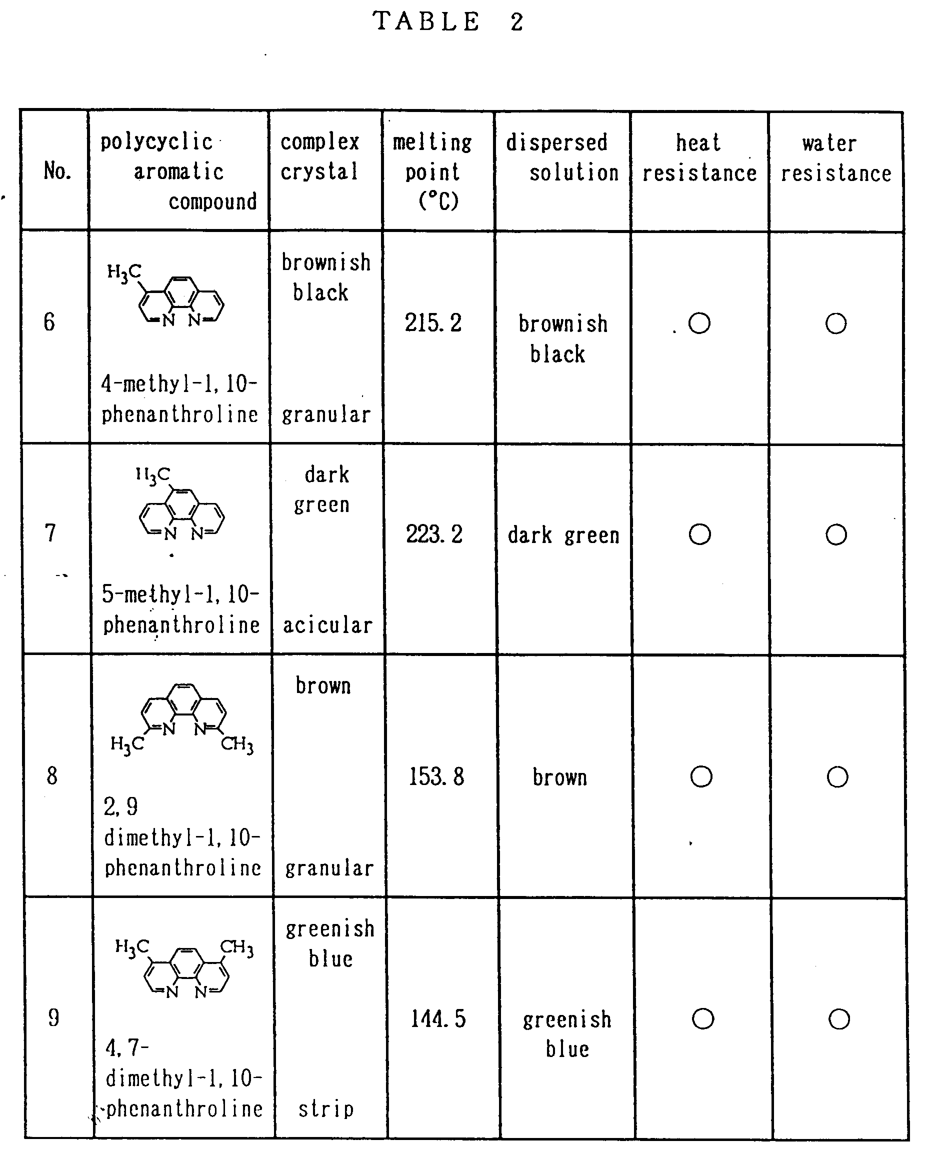

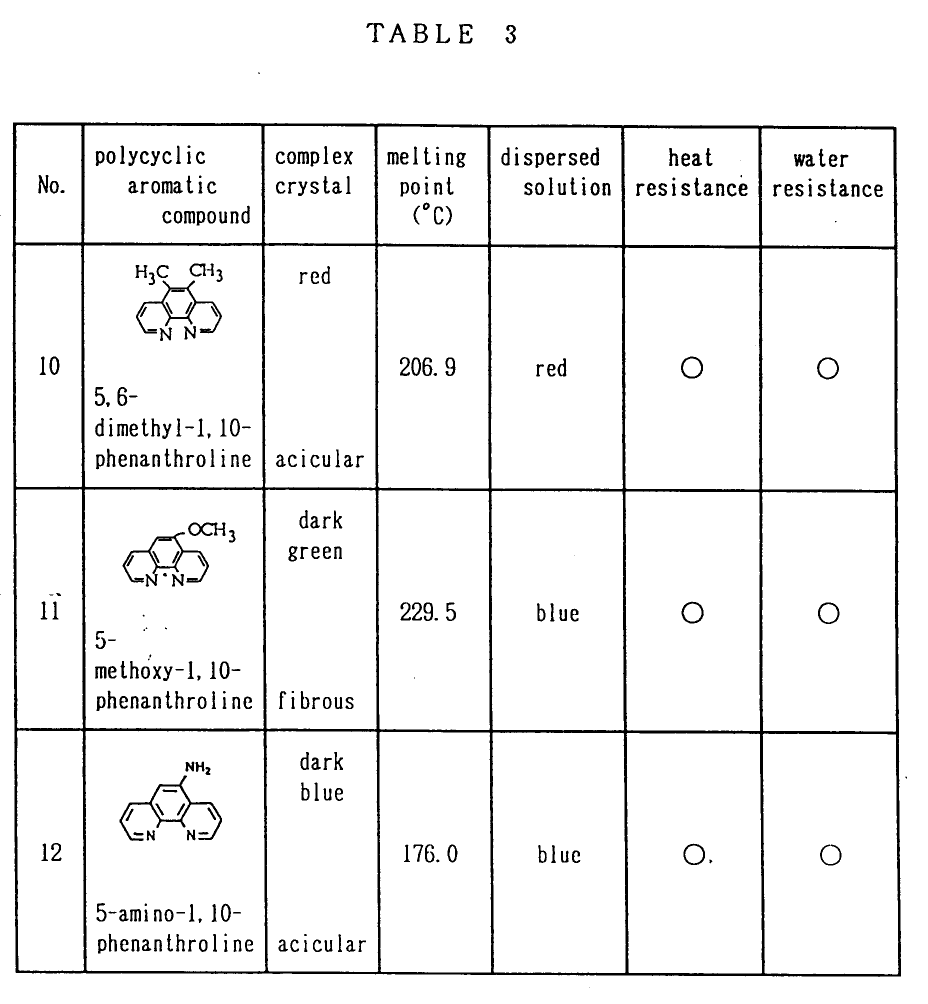

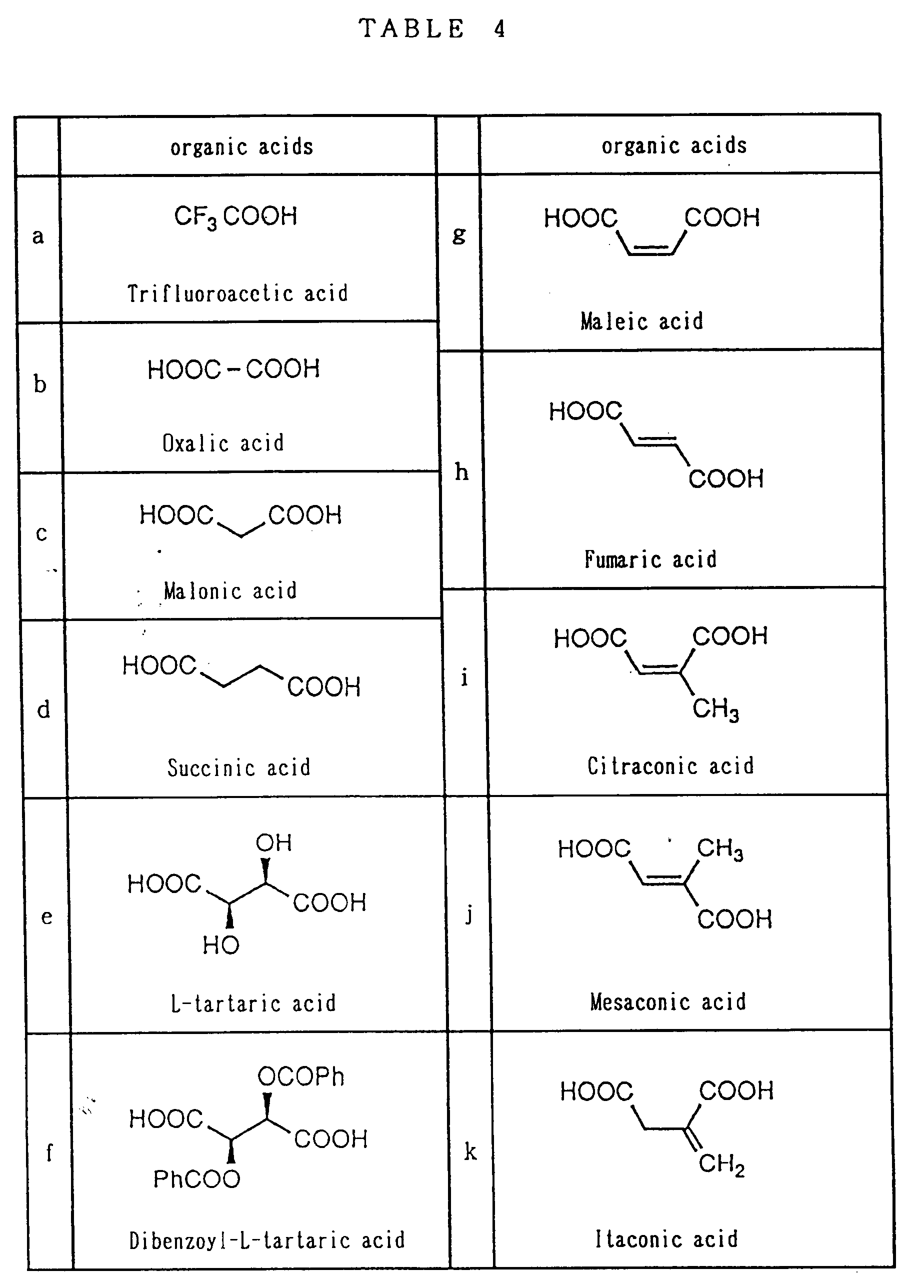

**TABLE 5**

| organic acids | added amount (g) | yield (g) | color | shape |
|---|---|---|---|---|
| a | 0.318 | 1.42 | brownish green | acicular + granular |
| b | 0.126 | 1.69 | dark green | acicular |
| c | 0.145 | 1.18 | dark green | acicular partly fibrous |
| d | 0.165 | 1.36 | dark green | acicular |
| e | 0.210 | 2.11 | dark green | acicular |
| f | 0.500 | 1.52 | dark green | acicular |
| g | 0.162 | 3.67 | dark green | acicular + fibrous |
| h | 0.162 | 2.16 | dark green | short acicular |
| i | 0.182 | 1.84 | greenish blue | acicular |
| j | 0.182 | 2.98 | dark green | granular |
| k | 0.182 | 1.82 | dark green | acicular |

**TABLE 6**

| No. of Embod. | composition of elements of complex crystals (wt%) | | | | | | composition ratio of complex crystals (polycyclic aromatic compound : HI₃ : H₂SO₄) |
|---|---|---|---|---|---|---|---|
| | C | H | N | O | S | I | |
| 1 | 34.96 | 2.06 | 6.67 | 2.78 | 1.03 | 52.50 | 7 : 4 : 1 |
| 2 | 38.85 | 2.24 | 7.41 | 0.76 | 0.28 | 50.46 | 2 : 1 : 0.08 |
| 4 | 38.17 | 2.47 | 3.28 | 5.00 | 1.93 | 49.15 | 4 : 2 : 1 |
| 6 | 38.69 | 2.60 | 6.92 | 0.96 | 0.17 | 50.66 | 2 : 1 : 0.04 |
| 7 | 41.03 | 2.74 | 7.37 | 0.82 | 0.19 | 47.85 | 2 : 1 : 0.05 |
| 11 | 41.37 | 2.77 | 7.36 | 4.89 | 0.17 | 43.44 | 7 : 3 : 0.05 |

The dispersed liquid obtained in the Second Preferred Embodiment in which the complex crystal comprising the 1,10-phenanthroline periodate compound was dispersed into ditridecyl phthalate was sealed in a cell to evaluate polarizability.

The evaluation cell included a pair of glass substrates 2 (made of soda-lime glass having a thickness of 1.1mm) which were disposed parallelly and opposedly each other, a pair of ITO (indium-tin oxide) transparent electrodes 3 which were formed respectively on opposing surfaces of the pair of glass substrates 2 in a thickness of 1500A, and an epoxy adhesive 4 which sealed circumferential portions of the pair of glass substrates 2. A dispersion medium 5 and the present light-adjusting particles of a complex crystal 1 were filled into a 100-micrometer-thickness cell gap which was surrounded by the pair of glass substrates 2 and the epoxy adhesive 4.

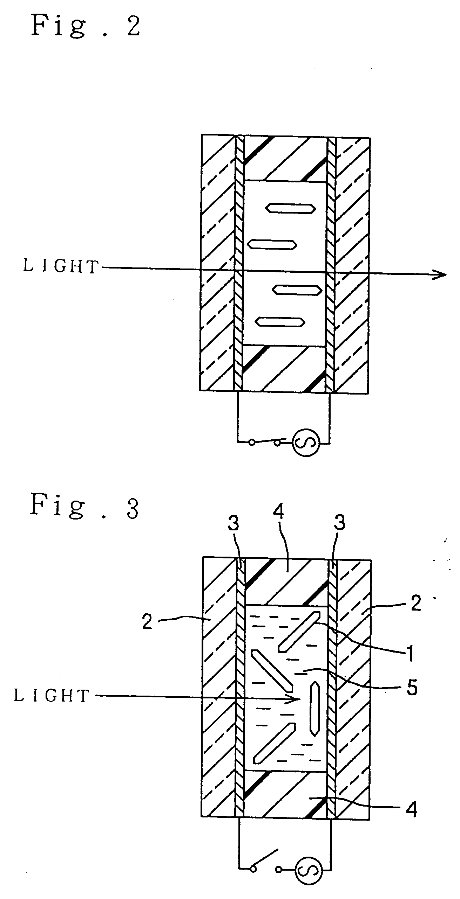

The above-described dispersed medium according to the Second Preferred Embodiment was sealed into the evaluation cell. As shown in Figure 2, when an electric field was applied on the dispersed medium, the light-adjusting particle 1 was oriented in a vertical direction against the glass substrates 2. Therefore, a light was transmitted through the evaluation cell, and light transparencies improved. On the contrary, as shown in Figure 3, when the electric field was not applied on the dispersed medium, the light-adjusting particle 1 was randomized and an irregular reflection of a light occurred. Therefore, a light was hardly transmitted through the evaluation cell, and light transparencies deteriorated.

As shown in Figure 1, the relationships between light transparencies and wave lengths was represented as a spectrum in a line graph. An axis of ordinate represented light transparencies, and an axis of abscissa represented wave lengths. Figure 1 showed three spectra: one was a spectrum when an electric field was applied (ON), another was a spectrum when an electric field was not applied (OFF), the other was a difference spectra between one spectra and another spectra. When an electric field (100V, 1kHz) was applied, the value of light transparencies showed about 50% in the range of 450 to 800nm. When the electric field was not applied, the value of light transparencies showed about 5%. When the applied voltage was varied in the range of 30 to 100V, the value of light transparencies was not changed. Therefore, the value of the difference spectra showed about 50% when the applied voltage was in the range of 30 to 100V. Thus, the complex crystal according to the second Preferred Embodiment showed a big difference spectrum, so it was suitable for use as light-adjusting particles for a light-polarizing glass.

The present invention discloses a complex crystal of polycyclic aromatic periodate compound which is formed by using a polycyclic aromatic compound containing at least one nitrogen atom and a polycyclic aromatic compound in which 3 rings are condensed. Since the complex crystal contains the polycyclic aromatic compound, a long conjugate system is formed, and the complex crystal has polarization. The complex crystal also has polarizability by means of a molecular chain of iodine. Furthermore, since the polycyclic aromatic compound has a facial structure, it stabilizes a structure of the complex crystal, and the complex crystal improves its heat stability. The complex crystal shows excellent moisture resisting property because a large facial expansion of the polycyclic aromatic compound protects iodine from an attack of a molecule of water. Moreover, the complex crystal exhibits excellent heat resistance since a substituent having an electron donativity is combined with the aromatic ring, and it stabilizes the aromatic ring.

The crystal according to the present invention is acicular or planar. As above-described, it also has polarization, so it is possible to control an orientation of particles of the complex crystal by means of an electric field. Therefore, the complex crystal of the present invention is suitable for use as light-adjusting particles.

A complex crystal is composed of an anion of triiodine and a cation of a polycyclic aromatic compound containing at least one nitrogen atom and 3 aromatic condensed rings and further contains an anion of an acid.

Since the complex crystal has such a stable structure, it shows excellent heat resistance and excellent moisture resisting property. Furthermore, the complex crystal has light-polarizing performance because of an arrangement of the molecular chain of iodine. Moreover, the complex crystal exhibits excellent polarization because of an interaction between the polycyclic aromatic compound and iodine. Therefore, the complex crystal is suitable for use as light-adjusting particles of a light valve or a light-adjusting glass.

## Claims

1. A complex crystal comprising an anion of triiodine and a cation of a polycyclic aromatic compound containing at least one nitrogen atom and three aromatic condensed rings optionally containing a substituent selected from amino, amide, hydrazide, imino or guanidyl as well as selected from hydrocarbon, aromatic hydrocarbon or their combination by way of sulfur or oxygen atom, provided that the substituent has a number of carbon atoms of not more than 10 and said complex crystal further containing an anion of an acid.

2. A complex crystal according to claim 1, wherein said polycyclic aromatic compound is acridine, phenanthridine, 1,7-phenanthroline, 1,10-phenanthroline, 4,7-phenanthroline, phenazine, phenoxazine, phenothiazine, carbazole, or imino stilbene.

3. A complex crystal according to claim 1, wherein said acid is an inorganic acid.

4. A complex crystal according to claim 3, wherein said inorganic acid is sulfuric acid.

5. A complex crystal according to claim 1, wherein said acid is an organic acid.

6. A complex crystal according to claim 5, wherein said organic acid is at least one selected from the group consisting of trifluoroacetic acid, oxalic acid, malonic acid, succinic acid, L-tartaric acid, dibenzoyl-L-tartaric acid, maleic acid, fumaric acid, citraconic acid, mesaconic acid and itaconic acid.

7. A complex crystal according to claim 1, wherein said acid is at least one selected from the group consisting of hydrochloric acid, phosphoric acid, hydriodic acid, benzenesulfonic acid, toluenesulfonic acid, methanesulfonic acid, acetic acid, propionic acid, butylic acid, baleic acid, benzoic acid, malonic acid and phthalic acid.

8. A complex crystal according to claim 1, wherein the melting point of said complex crystal is in the range of 140 to 230°C.

9. A complex crystal according to claim 1, wherein the color of said complex crystal shows no change when the dispersed liquid of said complex is heated at a temperature of 130°C for 10 hours.

10. A complex crystal according to claim 1, wherein the color of said complex crystal shows no change when said complex crystal is mixed with water.

11. A complex crystal accoding to claim 1, wherein said complex crystal is composed of 1,7-phenanthroline periodate compound which consists essentially of cation of 1,7-phenanthroline, cation of sulfuric acid and anion of triiodine, and the molar ratio of said 1,7-phenanthroline, said triiodine and said sulfuric acid is 7:4:1.

12. A complex crystal according to claim 1, wherein said complex crystal is composed of 1,10-phenanthroline periodate compound which consists essentially of cation of 1,10-phenenthroline and anion of triiodine, and the molar ratio of said 1,10-phenanthroline and said triiodine is 2:1.

13. A complex crystal according to claim 1, wherein said complex crystal is composed of 4,7-phenanthroline periodate compound.

14. A complex crystal according to claim 1, wherein said complex crystal is composed of acridine periodate compound which consists essentially of cation of acridine, cation of sulfuric acid and anion of triiodine, and the molar ratio of said acridine, said triiodine and said sulfuric acid is 4:2:1.

15. A complex crystal according to claim 1, wherein said complex crystal is composed of phenanthridine periodate.

16. A complex crystal according to claim 1, wherein said complex crystal is composed of 4-methyl-1,10-phenanthroline periodate compound which consists essentially of cation of 4-methyl-1,10-phenanthroline and anion of triiodine, and the molar ratio of said 4-methyl-1,10-phenanthroline and said triiodine is 2:1.

17. A complex crystal according to claim 1, wherein said complex crystal is composed of 5-methyl-1,10-phenanthroline periodate compound which consists essentially of cation of 5-methyl-1,10-phenanthroline and anion of triiodine, and the molar ratio of said 5-methyl-1,10-phenanthroline and said triiodine is 2:1.

18. A complex crystal according to claim 1, wherein said complex crystal is composed of 2,9-dimethyl-1,10-phenanthroline periodate compound.

19. A complex crystal according to claim 1, wherein said complex crystal is composed of 4,7-dimethyl-1,10-phenanthroline periodate compound.

20. A complex crystal according to claim 1, wherein said complex crystal is composed of 5,6-dimethyl-1,10-phenanthroline periodate compound.

21. A complex crystal according to claim 1, wherein said complex crystal is composed of 5-methoxy-1,10-phenanthroline periodate compound which consists essentially of cation of 5-methoxy-1,10-phenanthroline and anion of triiodine, and the molar ratio of said 5-methoxy-1,10-phenanthroline and said triiodine is 7:3.

22. A complex crystal according to claim 1, wherein said complex crystal is composed of 5-amino-1,10-phenanthroline periodate compound.

23. A method for forming a complex crystal according to Claim 1 comprising the steps of:
preparing a first solution in which an acid and a polycyclic aromatic compound containing at least one nitrogen atom and at least 3 aromatic rings are dissolved,
preparing a second solution in which iodine is dissolved, and
mixing said first solution and said second solution.

## Patentansprüche

1. Komplexkristall, der ein Anion des Trijods und ein Kation einer polycyclischen aromatischen Verbindung enthält, die zumindest ein Stickstoffatom und drei aromatische kondensierte Ringe aufweist, die gegebenenfalls einen Substituenten aufweisen, der sowohl aus Amino, Amid, Hydrazid, Imino oder Guanidyl ausgewählt wird, als auch aus Halogen, aliphatischem Kohlenwasserstoff, aromatischem Kohlenwasserstoff, oder ihre Verbindung über ein Schwefel oder ein Sauerstoffatom, ausgewählt wird, mit der Maßgabe, daß der Substituent eine Anzahl von nicht mehr als 10 Kohlenstoffatomen aufweist und dieser Komplexkristall weiter ein Anion einer Säure enthält.

2. Komplexkristall nach Anspruch 1, worin die polycyclische aromatische Verbindung Acridin, Phenanthridin, 1,7-Phenanthrolin, 1,10-Phenanthrolin, 4,7-Phenanthrolin, Phenazin, Phenoxazin, Phenothiazin, Carbazol oder Iminostilben ist.

3. Komplexkristall nach Anspruch 1, worin die Säure eine anorganische Säure ist.

4. Komplexkristall nach Anspruch 3, worin die anorganische Säure Schwefelsäure ist.

5. Komplexkristall nach Anspruch 1, worin die Säure eine organische Säure ist.

6. Komplexkristall nach Anspruch 5, worin die organische Säure zumindest eine ist, die aus der Gruppe ausgewählt wird, die aus Trifluoressigsäure, Oxalsäure, Malonsäure, Bernsteinsäure, L-Weinsäure, Dibenzoyl-L-weinsäure, Maleinsäure, Fumarsäure, Citraconsäure, Mesaconsäure und Itaconsäure besteht.

7. Komplexkristall nach Anspruch 1, worin die Säure zumindest eine ist, die aus der Gruppe ausgewählt wird, die aus Salzsäure, Phosphorsäure, Jodwasserstoffsäure, Benzolsulfonsäure, Toluolsulfonsäure, Methansulfonsäure, Essigsäure, Propionsäure, Butylsäure, Baleicsäure, Benzoesäure, Malonsäure, Phthalsäure besteht.

8. Komplexkristall nach Anspruch 1, worin der Schmelzpunkt von diesem Komplexkristall in dem Bereich von 140 bis 230 °C liegt.

9. Komplexkristall nach Anspruch 1, worin die Farbe des Komplexkristalls keine Änderung zeigt, wenn die dispergierte Flüssigkeit von diesem Komplex auf eine Temperatur von 130 °C für 10 Stunden erwärmt wird.

10. Komplexkristall nach Anspruch 1, worin die Farbe des Komplexkristalls keine Änderung zeigt, wenn der Komplexkristall mit Wasser gemischt wird.

11. Komplexkristall nach Anspruch 1, worin der Komplexkristall aus einer 1,7-Phenanthrolinperjodatverbindung zusammengesetzt ist, die im wesentlichen aus einem Kation des 1,7 Phenanthrolin, einem Kation der Schwefelsäure und einem Anion des Trijods und das molare Verhältnis von dem 1,7-Phenanthrolin, dem Trijod und der Schwefelsäure 7 : 4 : 1 beträgt.

12. Komplexkristall nach Anspruch 1, worin der Komplexkristall aus einer 1,10- Phenanthrolinperjodatverbindung zusammengesetzt ist, die im wesentlichen aus einem Kation des 1,10- Phenanthrolin und einem Anion des Trijods und das molare Verhältnis von dem 1,10- Phenanthrolin, und dem Trijod 2 : 1 beträgt.

13. Komplexkristall nach Anspruch 1, worin der Komplexkristall aus einer 4,7- Phenanthrolinperjodatverbindung zusammengesetzt ist.

14. Komplexkristall nach Anspruch 1, worin der Komplexkristall aus einer Acridinperjodatverbindung zusammengesetzt ist, die im wesentlichen aus einem Kation des Acridin, einem Kation der Schwefelsäure und einem Anion des Trijods und das molare Verhältnis von dem Acridin, dem Trijod und der Schwefelsäure 4 : 2 : 1 beträgt.

15. Komplexkristall nach Anspruch 1, worin der Komplexkristall aus einem Phenanthridinperjodat zusammengesetzt ist.

16. Komplexkristall nach Anspruch 1, worin der Komplexkristall aus 4-Methyl-1,10- Phenanthrolinperjodatverbindung zusammengesetzt ist, die im wesentlichen aus einem Kation des 4-Methyl-1,10- Phenanthrolin und einem Anion des Trijods und das molare Verhältnis von dem 4-Methyl-1,10- Phenanthrolin und dem Trijod 2 : 1 beträgt.

17. Komplexkristall nach Anspruch 1, worin der Komplexkristall aus 5-Methyl-1,10- Phenanthrolinperjodatverbindung zusammengesetzt ist, die im wesentlichen aus einem Kation des 5-Methyl-1,10- Phenanthrolin und einem Anion des Trijods und das molare Verhältnis von dem 5-Methyl-1,10- Phenanthrolin und dem Trijod 2 : 1 beträgt.

18. Komplexkristall nach Anspruch 1, worin der Komplexkristall aus 2,9-Dimethyl-1,10- Phenanthrolinperjodatverbindung zusammengesetzt ist.

19. Komplexkristall nach Anspruch 1, worin der Komplexkristall aus 4,7-Dimethyl-1,10- Phenanthrolinperjodatverbindung zusammengesetzt ist.

20. Komplexkristall nach Anspruch 1, worin der Komplexkristall aus 5,6-Dimethyl-1,10- Phenanthrolinperjodatverbindung zusammengesetzt ist.

21. Komplexkristall nach Anspruch 1, worin der Komplexkristall aus 5-Methoxy-1,10- Phenanthrolinperjodatverbindung zusammengesetzt ist, die im wesentlichen aus einem Kation des 5-Methoxy-1,10- Phenanthrolin und einem Anion des Trijods und das molare Verhältnis von dem 5-Methoxy-1,10- Phenanthrolin und dem Trijod 7 : 3 beträgt.

22. Komplexkristall nach Anspruch 1, worin der Komplexkristall aus 5-Amino-1,10- Phenanthrolinperjodatverbindung zusammengesetzt ist.

23. Verfahren zur Bildung eines Komplexkristalls nach Anspruch 1 aufweisend die Schritte:
Herstellung einer ersten Lösung, bei der eine Säure und eine polycyclische aromatische Verbindung, die zumindest ein Stickstoffatom und zumindest 3 aromatische Ringe enthält, gelöst wird,
Herstellung einer zweiten Lösung in der Jod gelöst wird und die erste Lösung und die zweite Lösung gemischt werden.

## Revendications

1. Un cristal complexe comprenant un anion triiodé et un cation d'un dérivé aromatique polycyclique contenant au moins un atome d'azote et trois cycles aromatiques condensés comportant éventuellement un substituant choisi parmi amino, amide, hydrazide, imino ou guanidyle ainsi que choisi parmi halogène, hydrocarbure aliphatique, hydrocarbure aromatique ou leurs combinaisons par l'intermédiaire d'un atome de soufre ou d'oxygène à condition que le substituant ait un nombre d'atomes de carbone non supérieur à 10 et que ledit cristal complexe contienne en outre un anion d'un acide.

2. Un cristal complexe selon la revendication 1, dans lequel ledit composé aromatique polycyclique est l'acridine, la phénanthridine, la 1,7-phénanthroline, la 1,10-phénanthroline, la 4,7-phénanthroline, la phénazine, la phénoxazine, la phénothiazine, le carbazole ou l'imino stilbène.

3. Un cristal complexe selon la revendication 1, dans lequel ledit acide est un acide minéral.

4. Un cristal complexe selon la revendication 3, dans lequel ledit acide minéral est l'acide sulfurique.

5. Un cristal complexe selon la revendication 1, dans lequel ledit acide est un acide organique.

6. Un cristal complexe selon la revendication 5, dans lequel ledit acide organique est au moins un acide choisi dans le groupe consistant en acide trifluoroacétique, acide oxalique, acide malonique, acide succinique, acide L-tartrique, acide dibenzoyl-L-tartrique, acide maléique, acide fumarique, acide citraconique, acide mésaconique et acide itaconique.

7. Un cristal complexe selon la revendication 1, dans lequel ledit acide est au moins un acide choisi dans le groupe consistant en acide chlorhydrique, acide phosphorique, acide hydriodique, acide benzènesulfonique, acide toluènesulfonique, acide méthanesulfonique, acide acétique, acide propionique, acide butylique, acide maléique, acide benzoïque, acide malonique et acide phtalique.

8. Un cristal complexe selon la revendication 1, dans lequel le point de fusion dudit cristal complexe est dans la fourchette de 140 à 230°C.

9. Un cristal complexe selon la revendication 1, dans lequel la couleur dudit cristal complexe ne varie pas lorsqu'une dispersion liquide dudit complexe est chauffée à une température de 130°C pendant 10 heures.

10. Un cristal complexe selon la revendication 1, dans lequel la couleur dudit cristal complexe ne change pas lorsque ledit cristal complexe est mélangé à l'eau.

11. Un cristal complexe selon la revendication 1, dans lequel ledit cristal complexe est composé d'un dérivé périodé de 1,7-phénanthroline qui consiste essentiellement en cation de 1,7-phénanthroline, cation de l'acide sulfurique et anion triiodé, et dont le rapport molaire de ladite 1,7-phénanthroline audit triiodé et audit acide sulfurique est de 7/4/1.

12. Un cristal complexe selon la revendication 1, dans lequel ledit cristal complexe est composé d'un dérivé périodé de 1,10-phénanthroline qui consiste essentiellement en un cation de 1,10-phénanthroline et d'un anion triiodé, et dont le rapport molaire de ladite 1,10-phénanthroline audit triiodé est de 2/1.

13. Un cristal complexe selon la revendication 1, dans lequel ledit cristal complexe est composé d'un dérivé périodé de 4,7- phénanthroline.

14. Un cristal complexe selon la revendication 1, dans lequel ledit cristal complexe est composé d'un dérivé périodé d'acridine qui consiste essentiellement en un cation d'acridine, un cation d'acide sulfurique et un anion triiodé, et dont le rapport molaire de ladite acridine audit triiodé et audit acide sulfurique est de 4/2/1.

15. Un cristal complexe selon la revendication 1, dans lequel ledit cristal complexe est composé de phénanthridine périodée.

16. Un cristal complexe selon la revendication 1, dans lequel ledit cristal complexe est composé d'un dérivé périodé de 4-méthyl-1,10-phénanthroline qui consiste essentiellement en un cation de 4-méthyl-1,10-phénanthroline et un anion triiodé, et dont le rapport molaire de ladite 4-méthyl-1,10-phénanthroline audit triiodé est de 2/1.

17. Un cristal complexe selon la revendication 1, dans lequel ledit cristal complexe est composé d'un dérivé périodé de 5-méthyl-1,10-phénanthroline qui consiste essentiellement en un cation de 5-méthyl-1,10-phénanthroline et un anion triiodé, et dont le rapport molaire de ladite 5-méthyl-1,10-phénanthroline audit triiodé est de 2/1.

18. Un cristal complexe selon la revendication 1, dans lequel ledit cristal complexe est composé d'un dérivé périodé de 2,9-diméthyl-1,10-phénanthroline.

19. Un cristal complexe selon la revendication 1, dans lequel ledit cristal complexe est composé d'un dérivé périodé de 4,7-diméthyl-1,10-phénanthroline.

20. Un cristal complexe selon la revendication 1, dans lequel ledit cristal complexe est composé d'un dérivé périodé de 5,6-diméthyl-1,10-phénanthroline.

21. Un cristal complexe selon la revendication 1, dans lequel ledit cristal complexe est composé d'un dérivé périodé de 5-méthoxy-1,10-phénanthroline qui consiste essentiellement en un cation de 5-méthoxy-1,10-phénanthroline et un anion triiodé, et dont le rapport molaire de ladite 5-méthoxy-1,10-phénanthroline audit triiodé est de 7/3.

22. Un cristal complexe selon la revendication 1, dans lequel ledit cristal complexe est composé d'un dérivé périodé de 5-amino-1,10-phénanthroline.

23. Un procédé de formation d'un cristal complexe selon la revendication 1, comprenant les étapes de :
- préparation d'une première solution dans laquelle on dissout un acide et un dérivé aromatique polycyclique comportant au moins un atome d'azote et au moins 3 cycles aromatiques,
- préparation d'une seconde solution dans laquelle on dissout de l'iode, et
- mélange de ladite première solution et de ladite seconde solution.
